# EUROPEAN PATENT APPLICATION

(11) **EP 1 281 409 A1**
(43) Date of publication of application: **05.02.2003**
(21) Application number: 02078030.0
(22) Date of filing: 24.07.2002
(51) Int. Cl.: A61M 5/178, A61M 5/24, A61M 5/28, A61J 1/00

(54) **Multifunctional device for administering medicinal substances**

(30) Priority: 31.07.2001 IT MI20011666
(71) Applicant: INGE S.p.A., 20024 Garbagnate Milanese (IT)
(72) Inventor: Nobbio, Alessio, 20123 Milan (IT)
(74) Representative: Coppo, Alessandro

(57) **Abstract**

A multifunctional device administering medicinal substances, comprising a cannula (10), suitable for administering a medical product (15), and a container and separator of substances (20), capable of being applied to one of the ends (14) of said cannula (10). The cannula (10) comprises a cylinder or tube (11) for containing the medical product and a thrusting piston (12) which can move, through a shaft (13), inside the tube (11), whereas the container-separator of substances (20) comprises a capsule (21) made of deformable material and a separator element (22), shaped as a regulator, suitable for closing the concave part of the capsule (21), so as to avoid the mixing of a liquid, powdered or granulated solute, contained therein, with the solvent consisting of the medical product (15), before the application of the compound. The mixing of the solvent and the solute takes place by deforming the capsule (21) and removing the separator element (22).

## Description

The present invention refers to a multifunctional device for administering medicinal substances and, more specifically, for supplying solvent substances to be associated, before application, with other pharmaceutical compounds comprising at least one liquid, powdered or granulated solute.

Currently, confections of medical products, in particular those for vaginal, anal or haemorrhoidal use, usually comprise a container for the medicinal substance to be applied and a series of cannulas which can be used for application.

Traditional cannulas comprise a collection tube, inside of which a piston integral with an actuation shaft can slide.

The tube usually has a threaded end so as to allow the container for the medicinal substance to be associated with it by screwing it in.

The opposite end is perforated to allow the partial removal of the actuation shaft from the tube, so that the cannula can be used correctly, firstly taking back the shaft and drawing the product into the tube and, then, ejecting the medical product in correspondence with the threaded end, being pushed by the flat surface of the piston, when it is made to slide in the opposite direction to the one for drawing in the product, through the actuation shaft.

Regarding this, the piston is possibly shaped and has a diameter which is greater with respect to the diameter of the actuation shaft and which is substantially equal to the inner diameter of the tube, in such a way as to slide with a certain friction along the inner walls of the tube, to guarantee the seal during the axial movement of the shaft, in the product application step, and the push outwards of most of the product previously drawn in, without said product filtering beyond the collection surfaces of the piston.

These known cannulas, however, have numerous drawbacks, such as constructive difficulties and substantially high production costs, due to the special shaping of the piston foreseen in correspondence with the end of the actuation shaft.

Moreover, for reasons of hygiene, it is absolutely necessary to foresee the realisation of disposable tubes and shafts and this determines a substantial consumption of material and a substantial increase in cost, according to the number of applications foreseen.

Finally, cannulas of this type cannot be used to administer solvent substances, such as medical products, to be mixed, before application, with a liquid, powdered or granulated solute, packaged separately from the solvent; regarding this, it should be remembered that keeping the solvent and solute of a pharmaceutical compound separate allows the lifetime of certain compounds to be increased, whereas said compounds, if packaged ready-to-use, would be very chemically unstable.

In this last case, it is furthermore possible that inexperienced people could remove the confection ignoring the content thereof; this happens above all when the solute is contained in particularly small amounts to the point of being practically invisible from the outside of the confection.

It is clear that in such a case taking such medicines can only produce psychological and therefore not therapeutic effects.

The purpose of the present invention is therefore that of avoiding the aforementioned drawbacks, realising a multifunctional device for administering medicinal substances, which allows the filling operations of the administering tube with specific medicinal substances to be eased, at the same time guaranteeing extremely low overall production costs, with respect to the prior art.

Another purpose of the present invention is that of realising a multifunctional device for administering medicinal substances which allows the administering of solvent medicinal substances to be mixed, before application, with specific liquid, powdered or granulated products, capable of being conserved, inside containers, separately from the aforementioned diluting solvent substances.

A further purpose of the present invention is that of realising a multifunctional device for administering medicinal substances which prevents the administering of the liquid, powdered or granulated solute, separate from the solvent, during the application of the medicinal substance.

These and other purposes, according to the present invention, are achieved by realising a multifunctional device for administering medicinal substances, according to claim 1, to which we refer for the sake of brevity.

Advantageously, according to the invention, it is possible to limit to the greatest possible degree the amount of material losses in each product application, consequently reducing the overall production costs of the pharmaceutical products.

Moreover, the application of a container is foreseen, arranged in correspondence with the outlet mouth of a cannula for administering medicinal unguents, to administer solvent substances (for example gel) to be mixed, before dispensing, with at least one solute to be diluted; the container is realised in such a way as to make the removal of a closing element impossible and, therefore, to make it impossible to open it, in the case in which a separator element of solvent and solute has not previously been removed from inside a capsule.

In such a way it is guaranteed that the liquid, powdered or granulated solute and the solvent are mixed like they need to be, by effect of gravity, before opening the container itself and administering the medical substance.

Further characteristics and advantages of a multifunctional device for administering medicinal substances, according to the present invention, shall become clearer from the following description, given as an example and not for limiting purposes, referring to the attached schematic drawings, in which:

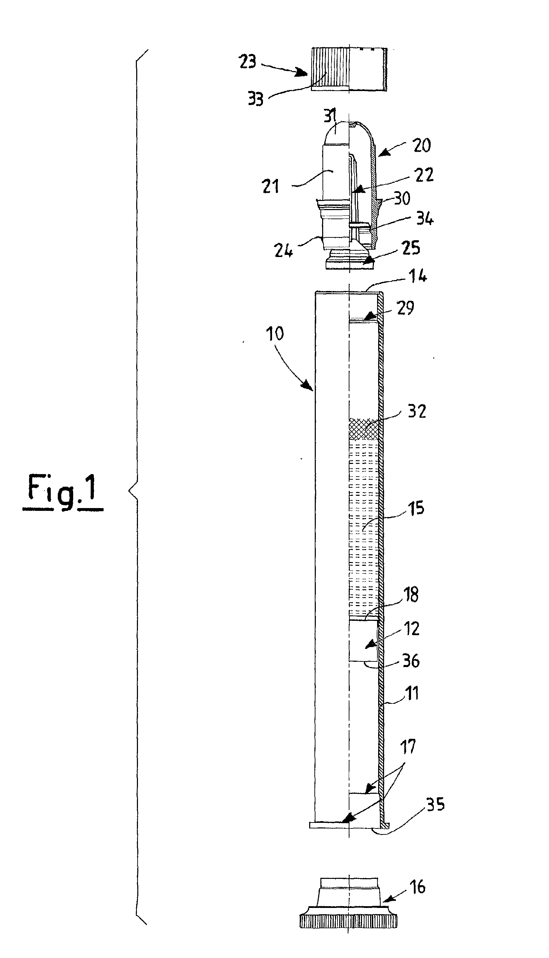

- figure 1 is an exploded view of a multifunctional device for administering medicinal substances, according to the present invention;

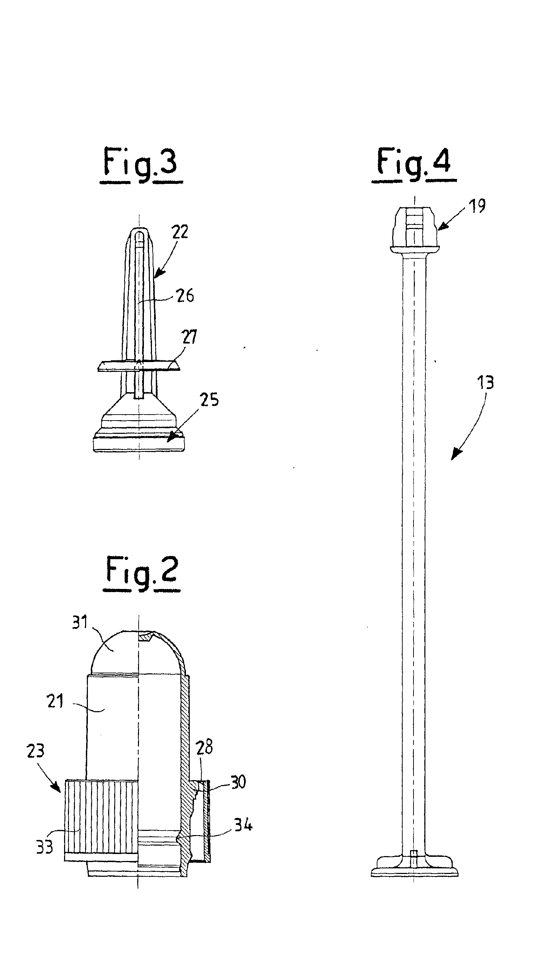

- figures 2, 3 and 4 represent a series of views of respective elements making up the multifunctional device for administering medicinal substances, according to the present invention;
figures 5 and 6 show two operative steps of use of the multifunctional device for administering medicinal substances, according to the invention.

With reference to the aforementioned figures, a cannula for administering solvent medicinal substances 15, such as gel or similar products, is generally indicated with 10; the cannula 10 comprises a tube or cylinder 11, open at the two ends, inside of which a small thrusting piston 12 can slide, capable of being actuated by a user from outside of the cannula 10, through a shaft 13.

A first end 14 of the tube 11 can carry a threading to allow a container for medicinal substances to be associated with it and to proceed to the insertion of the substance inside the cannula 10.

In preferred but not limiting embodiments it is, moreover, possible to realise a pre-assembled cannula 10, either filled or not filled with the medicinal substance 15 in the appropriate dose, inside of which is arranged the piston 12.

At the opposite end with respect to the one used to insert the solvent substance 15, the insertion of a suitably shaped closing cap 16 is foreseen in order to avoid the dispersion of the medical product.

In correspondence with the entry portion of the tube 11 some undercuts 17 are realised, as elements for stopping the piston 12, to avoid an undesired slipping out thereof, and for guiding the cap 16.

The undercuts 17 are realised through a raised edge, inside the tube 11, which extends along at least a circumferential arc thereof and can engage with a protruding upper ring 18 of the thrusting piston 12.

In correspondence with the end of the tube 11 closed by the cap 16, during application, the actuation shaft 13 of the piston 12 is introduced, which is shaped at the end 19, in order to make contact with the lower surface of the piston 12.

In correspondence with the end of the tube 11 usually closed by the cap 16, such a tube 11 can have, for a certain portion of side surface, a greater diameter with respect to the rest of the tube and, precisely near to the variation in diameter, it is possible to furthermore foresee an abutment shoulder, at which the piston 12 is held, to avoid it from slipping out from the tube 11 during the moving of the cannula 10.

The tube 11, moreover, can be equipped, in correspondence with said abutment shoulder, with small air ejection slits (not illustrated in the figures), so that it is filled correctly with the medical solvent.

The thrusting piston 12 is substantially cylinder-shaped and comprises a protruding upper ring 18, which takes care of guaranteeing the seal of the piston-tube coupling during the moving of the piston 12 inside the cannula 10.

The piston 12, finally, is preferably made out of plastic material which can slide quickly to ease its translation inside the tube 11 and to simplify the operations of filling of the tube 11 and of administering the product.

In correspondence with the end 14 of the tube 11, opposite the one where the cap 16 is usually inserted, an element 20 for containing and separating substances is arranged, suitable in particular for keeping a liquid, powdered or granulated solute separate from the medical solvent 15, which must be mixed with the solute only just before application.

The pharmaceutical compound comprising solute and solvent can thus be applied to carry out specific treatments, for example of the vaginal, anal or anti-haemorrhoidal type.

The element 20 for containing and separating compounds generically comprises a closing capsule 21, usually made out of substantially flexible plastic material, and a separator element 22, arranged inside the capsule 21.

The element 20 is housed, by means of a safety seal 23, realised in the form of a ring nut 33 with a grooved surface, on the outlet mouth of the tube 11 corresponding to the end 14.

The capsule 21 comprises a series of projecting parts arranged on the inner surface and an outer projecting part 24, whereas the actual separator element 22 comprises a cap portion 25 and a rod 26, to which an annular element or regulator 27 is bound.

The safety seal 23, consisting, as already stated previously, of a ring nut 33 with a circular section, is bound to the capsule 21 by means of breakable staples 28, so that it can be broken in a predetermined manner.

Moreover, the ring nut 33 constituting the safety seal 23 has a length such as to cover, when the capsule 21 is mounted, its point of engagement on the outlet mouth corresponding with the end 14 of the tube 11, so that the aforementioned seal can carry out its safety function correctly.

The operation of the multifunctional device for administering medicinal substances, according to the present invention, is substantially the following.

The confections of solvent medical substances 15, suitable for being applied by means of suitable administration cannulas, usually comprise a number of tubes or cylinders 11, usually already pre-assembled (and with the relative thrusting piston 12 inside), equal to the number of applications foreseen.

Each confection, moreover, contains a single actuation shaft 13 and a container for medical solvent substances 15 in general and, in particular for gel with which the pre-assembled cannula 10 is to be filled; alternatively, the confection can have a series of capsules or medical aids each comprising a tube 11, ready filled with a suitable dose of solvent substance 15, as well as a single actuation shaft 13.

In this step, the flow of the medical solvent substance 15 progressively pushes the piston 12 downwards, which was initially associated in abutment with the undercut 29 of the end 14.

The packaging of the solute inside the element 20 for containing and separating substances, which can be carried out through automatic machines, usually foresees a step of filling the capsule 21 with the liquid, powdered or granulated solute, and closing said capsule through the suitably forced insertion of the separator element 22.

The cap portion 25 of the separator element 22, in this way, locks between a pair of first inner projecting parts, causing a radial dilation of the capsule 21 in correspondence with the end intended for being housed in the outlet mouth of the cannula 10, which corresponds to the end 14 of the tube 11.

To ease the insertion of the element 20 in the outlet mouth of the tube 11, the end 14 of the cannula 10 can have a bevel with a smaller outer diameter with respect to the inner one of the mouth.

The insertion comes to a halt when the mouth of the end 14 of the cannula 10 meets an abutment and the outer projecting part 24 of the capsule 21 is situated, by effect of the radial dilation of the capsule 21, in engagement with the plane of the undercut 29 of the tube 11 realising the locking of the element 20 on the cannula 10.

The staples 28 are arranged at the upper end of the flange 30, so that the ring nut 33 of the safety seal 23 completely covers the side surface of said flange 30.

The side surface of the flange 30 is stepped, so as to have progressively decreasing diameters from the top towards the bottom, and is therefore easy to grip, once the safety seal 23 is removed.

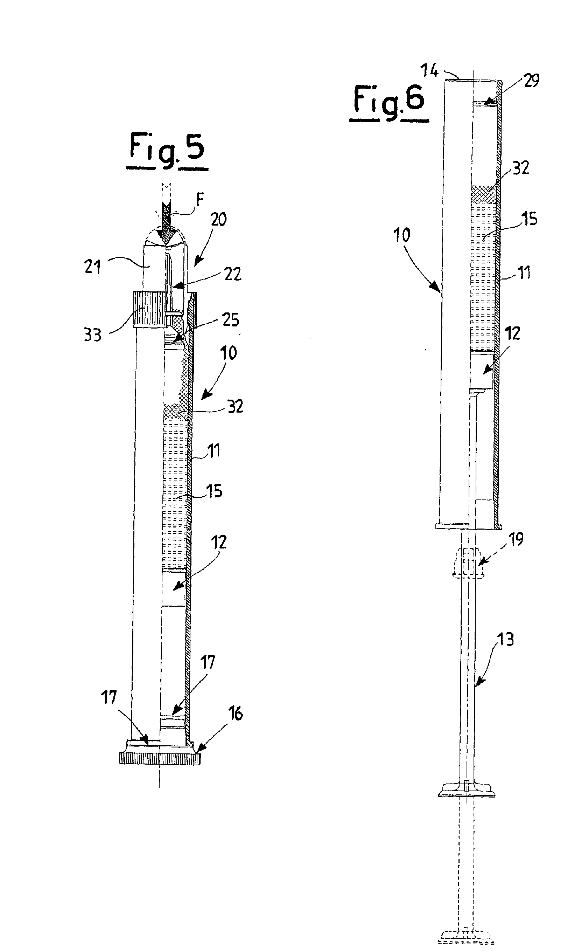

In the step of use of the multifunctional administering device, according to the invention, the separator element 22 is snapped by using pressure, along the direction of the arrow F (see figure 5), on the upper portion 31 of the capsule 21, with the consequent injection of the liquid, powdered or granulated solute, generically indicated with 32, into the tube 11, which mixes by gravity with the solvent substance 15.

In such a way, the regulator 27 stops on the first annular projecting part 34 preventing the separator element 22 from falling into the tube 11 of the cannula 10.

Therefore, the snapping of the staples 28 determines the breaking and the separation of the safety seal 23 from the element 20 and, in any case, reveals the indication of tampering or of attempted tampering of the administering device.

At this point, the elasticity of the capsule 21 determines the radial contraction of the portion previously engaged by the cap portion 25 of the separator element 22, whereas the outer projecting part 24 disengages from the plane of the undercut, unbinding the element 20 from the end 14 of the tube 11; therefore, only so long as the union of the solute 32 with the solvent substance 15 has taken place, the element 20 can be removed from the cannula 10.

Then, after having also manually removed the cap 16 from the end 35, opposite the end 14 of the cannula 10, one proceeds, as shown in figure 6, to insert the actuation shaft 13 into the tube 11, through the aforementioned end 35.

Next, to proceed to the application of the medicinal compound consisting of a solvent 15 and solute 32 substance it is sufficient to actuate the piston 12 through the shaft 13 which, in such a case, is removably connected to the piston 12 itself when, after having been introduced into the tube 11 from the side of the end 35, it is arranged in contact with the lower surface 36 opposite the annular projecting part 18.

Then, the shaped end 19 of the actuation shaft 13 is put into contact with the surface 36 of the piston 12 and each translation forward by a user from the outside translate into a thrust to the piston 12 to make it slide inside the tube 11, so as to compress and to make the medical compound for the desired application flow out from the side of the end 14.

It should be noted that the shaft 13 can take up an extremely simple configuration (a small elongated cylinder), so as to further contain the production costs, since it is sufficient that the shaped end surface 19 of the shaft 13 interacts with the lower surface 36 of the piston 12, in order to push the medical compound towards the outside.

In an alternative but not limiting embodiment of the device according to the invention the shaped end 19 of the actuation shaft 13 can have a configuration suitable for interacting against the inner walls of the tube 11, so that a portion of the side surface of the tube 11, having a shaped slit with a profile which matches that of the end 19, allows the shaft 13 to be introduced and removed laterally with respect to the tube 11, to then actuate it during the application of the medical compound.

From the description which has been made the characteristics of the multifunctional device for administering medicinal substances, which is object of the present invention, are clear, just as the advantages are also clear.

In particular, they are represented by:
- ease of filling the cannulas;
- reduction of material waste;
- possibility of reusing the same actuation shaft for many applications of a medicinal product;
- maintaining the hygienic conditions;
- possibility of keeping a solute separate from a medical solvent substance up to the act of application;
- low total production costs, with respect to devices of the traditional type.

Finally, it is clear that numerous variants can be brought to the multifunctional device in question, without for this reason straying from the novelty principles inherent to the inventive idea, just as it is clear that, in the practical embodiment of the invention, the materials, the shapes and the sizes of the illustrated details can be whatever according to requirements and they can be replaced with technically equivalent elements.

## Claims

1. Multifunctional device for administering medicinal substances, of the type comprising at least one cannula (10), which includes a tube (11), suitable for containing at least two medicinal substances (15, 32), and at least one piston (12) capable of moving inside said tube (11), **characterised in that** said piston (12) is removably connected to at least one moving shaft (13), capable of being actuated from outside of the tube (11), for the application of said medicinal substances, said shaft (13) being able to be introduced inside said tube (11) through a first end (35) of the tube (11).

2. Multifunctional device according to claim 1, **characterised in that**, on the opposite side with respect to said first end (35) and in correspondence with a second end (14) of the tube (11), the removable connection of at least one element (20) for containing and separating medicinal substances is foreseen.

3. Multifunctional device according to claim 2, **characterised in that** said element (20) for containing and separating allows the introduction of a solute (32) inside said tube (11), so that said solute (32) mixes with at least one medical solvent substance (15) contained in the tube (11), at a time immediately preceding the administering of the medical compound consisting of said mixture of solvent (15) and solute (32).

4. Multifunctional device according to claim 1, **characterised in that** said shaft (13) for moving the piston (12) comprises a shaped portion (19) at the end, which, in the step of administering the medicinal substance, comes into contact with at least one end surface portion (36) of said thrusting piston (12) so that said shaft (13) can push said piston (12) to make it slide inside said tube (11), so as to compress the medical substance and to eject it.

5. Multifunctional device according to claim 1, **characterised in that** said tube (11) of the cannula (10) has, in correspondence with at least said first end (35), at least one abutment undercut (17) for positioning said piston (12) and/or a closing element (16) for said first end (35).

6. Multifunctional device according to claim 2, **characterised in that**, in correspondence with said second end (14) of the tube (11), from which said medical compound comes out, at least one shoulder (29) is foreseen for positioning said element (20) for containing and separating.

7. Multifunctional device according to claim 2, **characterised in that** said element (20) for containing and separating medicinal substances comprises a capsule (21), made out of deformable material, and a separator means (22), suitable for closing the concave part of said capsule (21), through a cap portion (25), which engages with the inner surface of the capsule (21).

8. Multifunctional device according to claim 7, **characterised in that** the outer surface of said capsule (21) and the inner surface of said tube (11) carry mutual engagement means comprising at least one projecting part (24, 34) suitable for co-operating with undercuts, thanks to the radial expansion of the capsule (21), which takes place following the insertion of the cap portion (25) into the capsule (21).

9. Multifunctional device according to claim 7, **characterised in that** said separator means (22) comprises a rod (26) and a stopping regulator (27), arranged on said rod (26) and suitable for engaging at least one projecting part arranged on the inner surface of said capsule (21), so as to prevent the separator means (22) from falling inside said tube (11) of the cannula (10) in the act of mixing the solvent and the solute.

10. Multifunctional device according to claim 2, **characterised in that** said element (20) for containing and separating medicinal substances is applied on said tube (11) by means of a safety seal (23), of the type comprising a ring nut (33) with a circular section bound to said capsule (21) through a series of staples (28) which can be broken in a predetermined manner, arranged at an upper end of a flange (30) of said capsule (21).

11. Multifunctional device according to claim 10, **characterised in that** said flange (30) is made out of rigid material and has a stepped side surface with decreasing diameters from the top towards the bottom.

12. Multifunctional device according to claim 2, **characterised in that** said medicinal substances to be administered comprise medical compounds of the vaginal, anal or anti-haemorrhoidal type.
